# EUROPEAN PATENT SPECIFICATION

(11) **EP 3 427 787 B1**
(45) Date of publication and mention of the grant of the patent: **21.08.2019**
(21) Application number: 18192180.0
(22) Date of filing: 25.09.2014
(51) Int. Cl.: A61M 39/02, A61M 25/01, A61M 37/00

(54) **INDICIA BEARING SEPTUMS, METHODS OF MANUFACTURING THE SEPTUMS AND PORTAL THEREFOR**
MARKIERUNGSZEICHENTRAGENDE SEPTEN, VERFAHREN ZUR HERSTELLUNG DER SEPTEN UND PORTAL DAFÜR
SEPTUMS PORTEURS D'ÉLÉMENTS VISUELS, PROCÉDÉS DE FABRICATION DESDITS SEPTUMS ET PORTE ASSOCIÉE

(30) Priority: 08.10.2013 US 201361888047 P
(43) Date of publication of application: 16.01.2019
(62) Divisional of application: 14853048.8
(73) Proprietor: Smiths Medical ASD, Inc., Plymouth, MN 55442 (US)
(72) Inventor: FINBERG, Kristin, Minneapolis, Minnesota 55410 (US); HOOPLE, Cal Aaron, Hudson, Wisconsin 54016 (US); KUBAS, Amy, Hugo, Minnesota 55038 (US); TRAVIS, Ronald Gene, Spring Lake Park, Minnesota 55432 (US); WOO, Louis, Alexandria, Virginia 22314 (US)
(74) Representative: Hedges, Martin Nicholas

(56) References cited:
- WO-A2-2013/138813
- US-A1- 2011 257 609
- US-A1- 2012 078 202
- US-B1- 6 287 293

## Description

### Field of the Invention

The instant invention relates to implantable medical devices and more particularly to portals having an indicia bearing septum that can readily be identified after being implanted into a patient, and methods of manufacturing the indicia bearing septum.

### Background of the Invention

A port, or portal, is a medical device having a housing fitted with a resealable septum to provide a reservoir that is implantable subcutaneously in a patient so that the fluid stored in the reservoir may be directed, by means of a catheter attached to the output of the portal, to a particular location in a patient. To gain access to the reservoir, a cannula is inserted through the resealable elastomeric septum so that fluid may be input to or withdrawn from the reservoir.

US patent No. 8,092,435, assigned to the same assignee as the instant application, discloses a portal that has a septum embedded indicia. The septum has a base onto which a depression is formed on the top surface thereof, so that radiopaque material may be injected into the depression. A silicone layer then covers the top of the septum base to seal-in the hardened radiopaque material. That the radiopaque material needs to be injected in fluid form into the septum base is a time consuming process. The instant invention provides an improved indicia bearing septum(s) and a method(s) of manufacturing such indicia bearing septum with substantial time savings and improved efficiencies.

US 2012/078202, which is considered to represent the closest prior art, discloses a septum including a resealable elastomer septum base having a top surface, at least one depression of a given configuration formed at the top surface of said septum base, said depression viewable by a viewer as an information bearing indicia.

Other prior art arrangements are disclosed in US 6287293 and WO 2013/138813.

### Brief Summary of the Invention

According to the present invention there is provided a septum according to claim 1.

A septum according to the invention has the advantage that, by molding the radiopaque indicia, due to the compacting of the radiopaque material during the molding process, the resulting insert mounted to the septum base, when viewed under radiographic imaging, provides an improved visual representation than the indicia embedded septums disclosed in the aforenoted '435 patent.

The present invention is also directed to a port implantable into a patient according to claim 10.

### Brief Description of the Figures

The present invention will become apparent and the invention itself will be best understood with reference to the following description of the present invention taken in conjunction with the accompanying drawings, wherein:
Fig. 1 is a disassembled view of the subcutaneous implantable portal of the instant invention;
Fig. 2 is a perspective view of the septum of the instant invention that is a part of the portal shown in Fig. 1;
Fig. 3 is a plan view of the portal of Fig. 1;
Fig. 4 provides a perspective view of an assembled portal of the instant invention;
Fig. 5 is a disassembled view of the septum of the instant invention;
Fig. 6A is a perspective view of the assembled septum of the instant invention;
Fig. 6B is a plan view of the inventive septum of Fig. 6A;
Fig. 6C is a cross-sectional view of the inventive septum;
Fig. 7 illustrates the mold for manufacturing the radiopaque insert for the inventive septum;
Fig. 8 illustrates the mold for making of the septum base of the inventive septum;
Fig. 9 is an illustration of an alternative embodiment of the inventive septum; and
Fig. 10 is a cross-sectional view of the inventive septum shown in Fig. 9.

### Detailed Description of the Invention

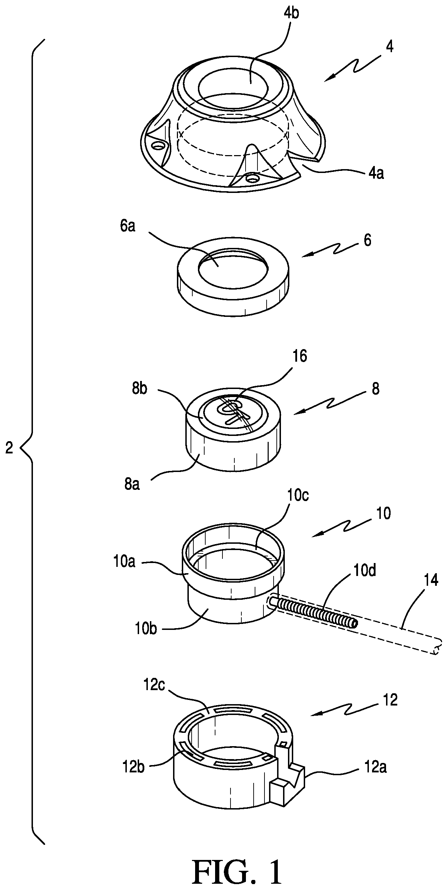

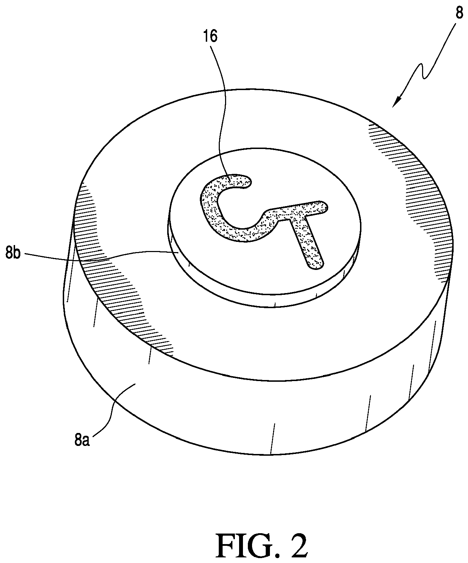

Fig. 1 is similar to Fig. 2 of the above-discussed US patent No. 8,092,435, the disclosure of which is incorporated by reference herein. The only difference is the septum for the portal or port 2 shown in the disassembled view. In brief, the subcutaneous implantable port 2 enables the port to be located once it is subcutaneously implanted into the patient, so that the fluid, for example a liquid medicament input and stored in the reservoir of the port for example by means of a pump or a syringe, can be output to a particular destination inside the patient. Alternatively, fluid in the reservoir may be withdrawn by using a syringe, for example. The port of the instant invention provides an identification of the port and the location of the port inside the patient, under radiographic imaging including x-ray and computer tomography imaging.

Fig. 1 shows a disassembled view of the various components or elements of the port of the instant invention. In particular, port 2 includes a housing 4, a cap 6, a septum 8, a reservoir body 10 and a housing base 12. Reservoir body 10 is cup-shaped and is shown to have an upper portion 10a and a lower portion 10b, which includes the base of the reservoir body 10. A shoulder 10c joins upper portion 10a to lower portion 10b. An outlet 10d extends from lower portion 10b of reservoir body 10. A conduit or catheter 14, in phantom line, is connected to outlet 10d for transporting the fluid stored in reservoir body 10 to a selected location within the patient, when port 2 is implanted subcutaneously in the patient.

Fitted to the upper portion 10a of reservoir body 10, with shoulder 10c providing a rest stop therefor, is septum 8. As shown in Fig. 1, septum 8 is a one-piece integral unitary component that has a septum base 8a and an elevated top 8b. In fact, septum 8 is made in multiple steps that will be described in detail, *infra.* An information bearing indicia element or insert 16 is embedded in septum 8. The process of embedding insert 16 in septum 8 will also be described in detail, *infra.*

Once fitted to upper portion 10a of reservoir body 10, to hold septum 8 in place, cap 6 is friction fitted over top portion 10a of reservoir body 10. For the exemplar port shown, both cap 6 and reservoir body 10 are made from plastic material, titanium or some other inert metal acceptable for implantation to a patient. An opening 6a at cap 6 exposes top layer 8b, and more particularly the information bearing indicia 16 embedded in septum 8. The assembled reservoir housing -- made up of reservoir body 10, septum 8 and cap 6 -- is then placed in housing base 12, which is in the form of a collar with its inside diameter having a dimension sufficient to receive reservoir body 10. A notched support 12a at a side of housing base 12 provides support to outlet 10d. Housing 4 then is positioned over housing base 12 to envelope the assembled reservoir housing. A slot 4a at the lower portion of housing 4 provides accommodation for outlet 10d extending out from reservoir body 10. A top opening 4b at housing 4 exposes the top surface of septum 8, and therefore the information bearing indicia embedded in septum 8. To prevent separation, housing 4 and housing base 12 are ultrasound welded, possibly at the location defined by grooves 12b at the lip 12c of housing base 12. Housing 4 and housing base 12 may be made from conventional medical plastics material to reduce the cost and the weight of the port.

Fig. 2 is a perspective view of septum 8 of the instant invention. Note that the information bearing indicia 16 is a one-piece insert that is embedded into the top surface and the upper portion of septum base 8a. The slightly upraised layer 8b of septum 8 may be considered to the upper portion of the septum. The finished exemplar septum 8 in Fig. 2, although not clearly shown, has a clear silicone layer formed over the top of the septum base to encapsulate and/or insulate the information bearing indicia insert 16 and the top of the septum from the environment. As shown, indicia 16 is a one continuous insert element that has a given configuration, shown in the exemplar septum 8 of Fig. 2 to be a conjoint "C" and "T". It should be appreciated however that other configurations may also be used as indicia 16, including symbols and non-alphanumeric characters. So, too, instead of one single continuous configuration, indicia 16 may be formed by multiple inserts that are separately provided in and viewable from septum 8.

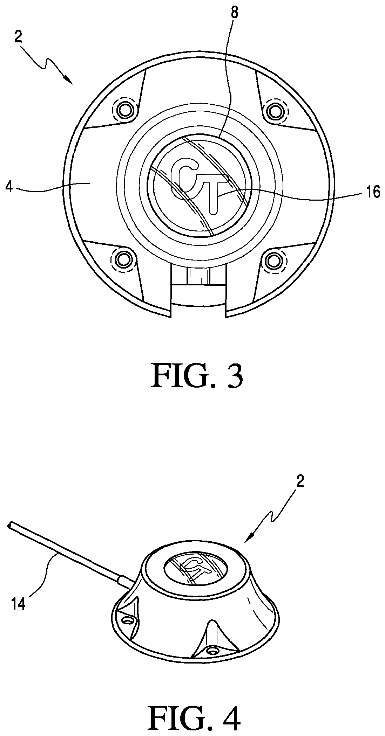

Fig. 3 is a plan view of the assembled port 2. Fig. 4 shows a perspective view of the assembled port 2 having a catheter 14 connected to its outlet.

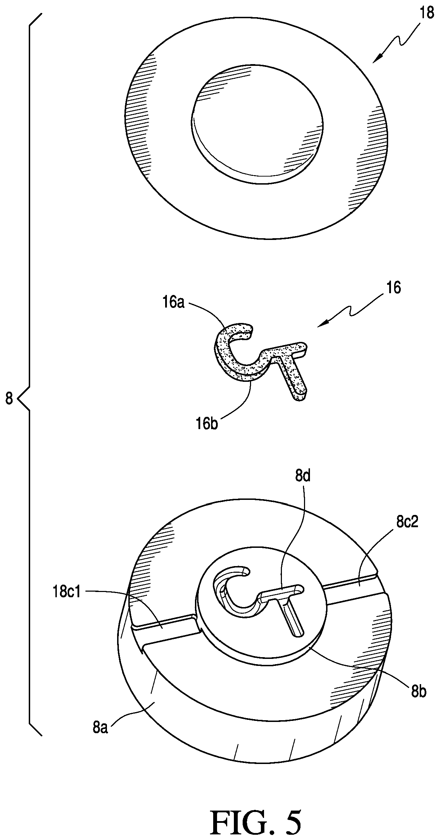

With reference to Fig. 5, a disassembled exemplar septum 8 of the instant invention is shown to have a septum base or puck 8a that has a raised layer 8b. Septum base 8a may be made from a silicone gum stock or LIM (liquid injection molding) material, for example. Base 8a is fabricated to have a thickness in a cross section to enable at least the lower portion of it to be form fitted into the upper portion 10a of reservoir housing 10. The bottom surface of septum base 8a may be flat. Two channels 8c1 and 8c2 are provided for the inflow and outflow, respectively, of liquid silicone to form the clear silicone layer, designated 18, that covers the top of the septum base to complete the manufacturing of the septum. As noted above, septum base 8a has a raised layer 8b where a particular impression, mark or indicia 8d of a given configuration is formed as a depression 8d. As shown, depression 8d is a continuous trench, groove or cavity that provides a readable information bearing indicia to a viewer. In the exemplar septum base 8a shown in Fig. 5, depression 8d is in the form of an alphanumeric information bearing indicia cavity that has combined and joined the letters "C" and "T". That the depression 8b is formed as one continuous trench, groove or cavity provides for easy assembly for the septum, as will be described below.

As disclosed in the aforenoted '435 patent, for the previous generation of septum, a liquid radiopaque material such as barium sulfate (BaSo4), or some other similar radiopaque material viewable under radiographic imaging, is injected into the depression 8d by using an injection mechanism such as a syringe. After the liquid radiopaque material is injected onto the septum, it has to be solidified and hardened, and cured, before anything else can be done. As a result, a waiting time is incurred in the manufacturing of the previous generation septum.

The septum of the instant invention eliminates this waiting time by providing a one-piece solid radiopaque insert 16 that fittingly mounts into the cavity formed by depression 8d in septum base 8a. As shown in Fig. 5, insert 16 has the same configuration as that formed by depression 8d on the top surface of septum base 8a. For ease of handling, and also to avoid confusion, the manufacturing process may be such that the cavity effected by depression 8d at septum base 8a has a curved internal trench surface, i.e., a groove. On the other hand, insert 16 is molded to have a flat top surface 16a but an outwardly curved under surface 16b so that insert 15 may be fittingly mounted into the cavity formed by depression 8d. After insert 16 is mounted into depression 8d, the top surface of septum base 8a, which may be flush with the top surface of the insert, is coated with a liquid silicone layer, identified as top layer 18, so that the radiopaque insert 16 and the top of the septum are encapsulated and therefore isolated from the environment.

With the embedding of the one piece solid insert 16 into the septum base 8a, since the radiopaque material is white whereas the septum base formed from silicone is transparent or translucent as is the silicone top layer 18, the clinician can readily visually view the indicia formed by insert 16 from septum 8. Moreover, the port described above manufactured with the exemplar septum shown in Fig. 5, when implanted to a patient, would provide a readable indicia under x-ray or computer tomography. It should moreover be understood that the readable indicia can be read to represent certain characteristics of the port, including for example whether the port is adapted to be used for power injection and the storage capacity of the port fluid reservoir.

As discussed above, for the prior generation septum manufactured under the '435 patent, a liquid radiopaque material has to be injected into the depression formed at the septum base. The process of injecting the liquid radiopaque material is tedious, takes a substantial amount of time and requires a great amount of quality control be in place to ensure that the liquid radiopaque material properly solidifies and cures. Moreover, blemishes or defects formed in the liquid injection radiopaque material is something that is not readily detectable. With the instant invention where a one-piece solid insert is directly mounted into the cavity formed by the depression at the septum base, a number of advantages are achieved. Foremost, the increase in manufacturing efficiency that results from the molding of a great number of inserts, so that the number of septums produced per hour under the manufacturing process of the instant invention increases at least by 20 to 30 fold. Further, there is an improvement in the quality control insofar as blemishes in an insert are readily detected when the insert is extracted from its mold. This ensures that the radiopaque insert is fully cured and is ready to be inserted into a corresponding septum base. Furthermore, there is a great deal of cost savings with the manufacturing of a one-piece solid insert due to the fact that the inserts are produced by molding, and each insert mold can contain multiple numbers of the inserts. Furthermore, that the radiopaque insert is formed by molding means that the liquid radiopaque material injected into the insert mold is compacted during the molding process to thereby provide a tightly compacted solid insert, which in turn provides a better and more clear view of the insert under radiographic imaging.

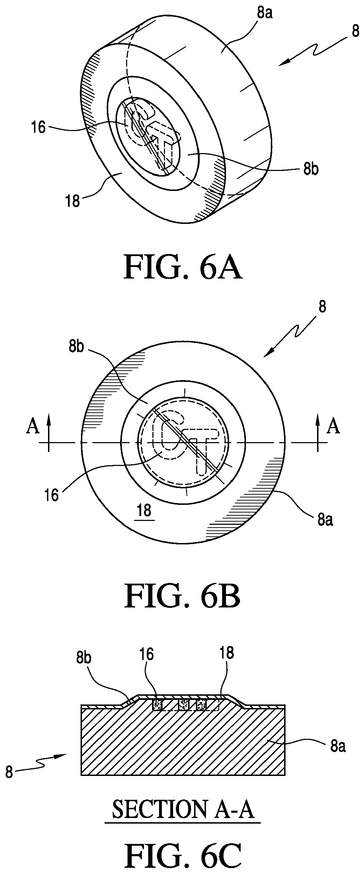

Fig. 6A shows an assembled inventive septum in a perspective view with the embedded radiopaque insert shown in dotted lines. Fig. 6B shows a plan view of inventive septum 8, and Fig. 6C is a cross-sectional view along section A--A of septum 8.

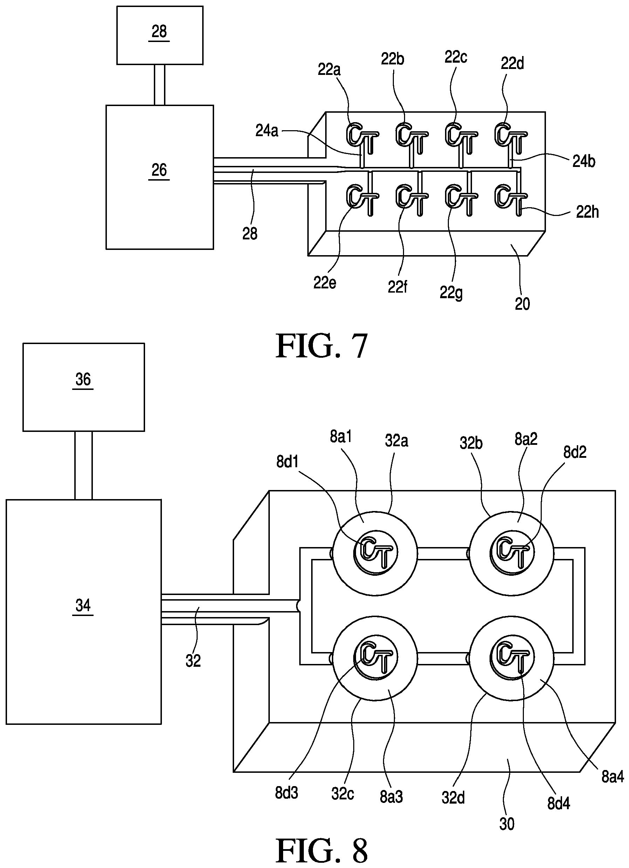

An exemplar process of manufacturing the radiopaque insert for the septum of the instant invention is shown in Fig. 7. As shown, an insert mold 20 is made to have a plurality of molding cavities, for example the eight cavities shown in the exemplar mold 20. There could be a smaller or a greater number of molding cavities 22 than that shown in Fig. 7. Each of the molding cavities 22 is formed in the shape of the given configuration of the insert 16, in this example the conjoint "CT" configuration, that corresponds to the configuration of the depressed impression formed on the septum base to which the insert is to be mounted. A fluid flow channel 24 connects mold 20 to a pump 26 that feeds a liquid radiopaque material, for example barium sulfate (BaSO4), from a liquid radiopaque material store 28 to mold 20. Channel 24 delivers the liquid radiographic material to its different branches, of which only branches 24a and 24b are labeled, so that the liquid radiopaque material fills each of the molding cavities 22a-22h. Mold 20 has a cover (not shown for sake of clarity) that covers mold 20 so that the liquid barium is injected into the different molding cavities and is compacted separately in each of the cavities, as is conventionally known in injection molding. With the cover removed, and the liquid radiopaque material having been solidified and hardened, the thus cured multiple one-piece solid inserts 22a-22h may be extracted or retrieved from the respective molding cavities of mold 20. Each of the extracted inserts is then mounted to the cavity that is formed by the depression at a corresponding septum base, for example 8d at septum base 8a, as described previously. Thus, with the manufacturing process as discussed above, a large number of radiopaque inserts may be molded for each batch of molding.

Fig. 8 shows a septum mold 30 wherein a plurality of septum cavities, for example the four cavities 32a-32d, are shown. For the sake of convenience, Fig. 8 shows only the base of the mold, with the top of the mold having been removed, and the four septum bases formed in the mold, so that the respective impressions formed by the corresponding depressions 8d are shown for the molded septum bases. For clarification purposes, the septum bases in Fig. 8 are labeled 8a1-8a4, with their respective depressions labeled 8d1-8d4. The thus formed septum bases may be extracted from the mold. Each of the septum bases then would have a corresponding radiopaque insert 16 mounted into the cavity formed by the depression at the top surface and the upper portion of the septum base. The liquid silicone stock to form the septum bases is injected to mold 30 by way of flow channel 32, with a pump 34 providing the liquid silicone from a liquid silicone store 36.

As discussed above, after a radiopaque insert having the same configuration as the impression created by the depression formed in the septum base is inserted into the cavity formed by the depression, a liquid layer of silicone is injected or placed over the top of the septum base, so that a clear silicone layer is bonded to the septum base to form the inventive septum. As was pointed out above, the respective top surfaces of the insert and the septum base may be flush with each other to ensure that the clear silicone layer covers both top surfaces to thereby provide an evenly distributed insulation layer.

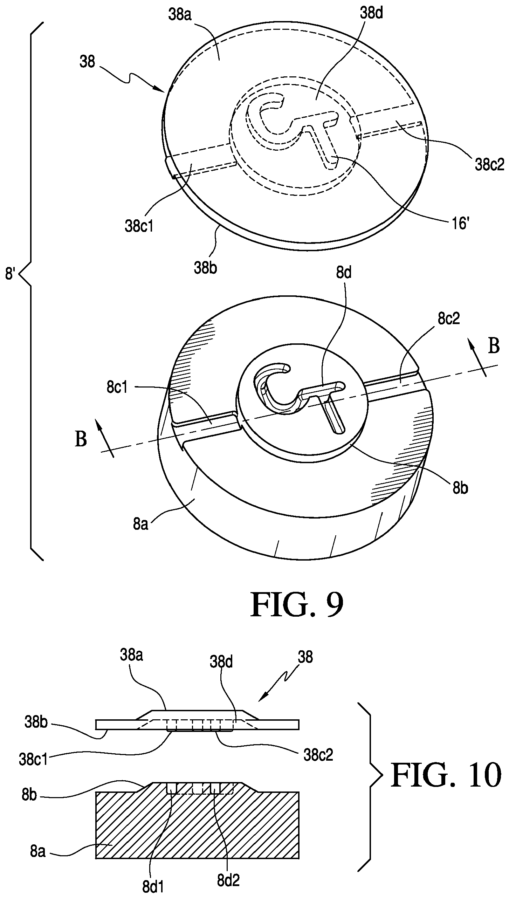

An alternative process for manufacturing the information bearing indicia embedded septum of the instant invention is illustrated in Figs. 9 and 10. For this alternative embodiment, the septum base is made in identical fashion as discussed in the earlier embodiment. Thus, the same reference numbers are used to identify the different parts of the septum base.

In the alternative embodiment, instead of a solid insert, a top layer with an integral insert is formed from a mold to have a configuration that form fits over the top of septum base 8a. Septum top 38 may be formed from a liquid silicone impregnated with a radiopaque material, for example barium sulfate (BaSO4), so that the complete top layer is radiopaque. As shown, top layer 38 has an upper or top surface 38a and a lower or bottom surface 38b that has hanging downwardly therefrom a number of legs or extensions that together form a downwardly extending leg formation or insert that has the same configuration as insert 16 shown in Fig. 5, for example the exemplar conjoint "CT" configuration. This downward extension or leg formation is labeled 16' in the septum top layer 38 shown in Fig. 9. There may also be two integral arms 38c1 and 38c2, shown in dotted lines, that hang downwardly from the bottom surface 38b of top layer 38. Downwardly extending arms 38c1 and 38c2 would fittingly mate to channels 8c1 and 8c2, respectively, formed at the top of septum base 8a. There is moreover a slightly concave cavity 38d, also shown in dotted line, that enables septum top 38 to formingly fit over the raised layer 8b at septum base 8a. For the exemplar septum 8' shown in Fig. 9, there is therefore only two components, i.e., the one-piece solid radiopaque septum top 38 form fitted to the top of septum base 8a. Once positioned and fitted onto septum base 8a, septum top 38 may be bonded to septum base 8a by a number of conventional known methods such as for example ultrasound bonding or gluing.

Fig. 10 shows a cross-sectional view B --B of the septum base 8a and the septum top 38 superposed thereover. As shown, legs 38c1 and 38c2 extending downwardly from the bottom surface 38b of septum cover 38 are the portions of the downward extending insert 16' that mount into the cavities 8d1 and 8d2 shown for the cross-sectional view of septum base 8a. For the septum base embodiment shown in Figs. 9 and 10, the depth of the depression 8d may be deeper than the depression for the embodiment shown in Fig. 5, so that once septum top 38 is bonded to septum base 8a, with the downward hanging inserts 16' fitted into the cavity formed by the depression 8d, the greater length of the insert 16' means that that portion of the radiopaque material can more readily be seen, and be differentiated from the rest of top layer 38, when the septum is view under radiographic imaging, even though septum top 38 is radiopaque.

There are advantages for utilizing the alternative embodiment of the one piece combination top and leg formation layer shown in Figs. 9 and 10. One advantage is that since septum top 10 is impregnated with radiopaque material, the top of the septum formed therewith would be just like other ports except that it is cloudy or white, depending on the radiopaque material used. That notwithstanding, a readable indicia nonetheless can be viewed under radiographic imaging. Further, with the alternative embodiment, the manufacturing of an information bearing indicia septum becomes easier insofar as both the septum base and the septum top that form the inventive indicia embedded septum are separately molded and put together in one single step, therefore resulting in greater efficiency and decreased cost. As is the case with the septum shown in Fig. 5, the septum shown in Figs. 9 and 10 is fitted into reservoir housing 10 shown in Fig. 1, during the assembly of the port 2 of the instant invention.

In place of the top layer insert separately formed at a different mold in the manner described above in Fig. 7, the combination top layer for the septum of the embodiment shown in Figs. 9-10 may be formed directly from the same mold that forms the septum base. For this manufacturing process, the top portion of the mold that covers the septum base has a cavity facing the cavity at the septum base portion of the mold so that the liquid silicone impregnated with the radiopaque material may be injected into the mold, and flow via the respective input channel 8c1 and out flow channel 8c2, after the formation of the septum bases. The injected liquid silicone would fill the impression cavity formed by depression 8d and bonds to the top surface of the septum base to form the one piece combination top and leg formation insert thereat. After the liquid top layer is cured and hardened, the thus formed one piece inventive septum may be removed from the mold.

It is also described a method of manufacturing an indicia bearing septum, comprising the steps of: (a) providing an elastomeric septum; (b) forming at least one depression of a given configuration at a top surface of the septum; (c) molding a radiopaque insert having the given configuration; and (d) mounting the radiopaque insert into the depression at the septum; wherein the insert provides an indicia to a viewer visually and when the septum is viewed under x-ray or computer tomography imaging.

The method may further comprise the step of: covering the top surface of the septum wherefrom the depression is formed with a transparent elastomeric layer after the insert has been mounted into the depression to insulate the insert from the environment. Step (c) may further comprise the steps of: configuring a mold to have a cavity shaped to form the insert having the given configuration; injecting a radiopaque material into the mold cavity; and solidifying the radiopaque material injected into the mold cavity to form the insert as a radiopaque one piece solid insert.

The injecting step optionally comprises the step of injecting liquid Barium Sulfate (BaSO4) into the mold cavity.

The method may further comprise the step of covering the top surface with a silicone layer. Step (b) may further comprise the step of forming the depression from the top surface of the septum to a predetermined depth into the septum. Alternatively, step (b) may further comprise the step of: forming the given configuration of the depression as a readable indicia that is adapted to convey information to a viewer.

An embodiment of the invention may further provide a port implantable into a patient, comprising: a housing having a chamber with an opening and an outlet, the opening having fitted therein a septum of the invention, the septum sealing the opening of the chamber to form a reservoir in the housing adapted to store a fluid, a catheter connected to the outlet to be in fluid communication with the reservoir so that the fluid is conveyable between the reservoir and the patient when the port is implanted into the patient; wherein, after the port is implanted in the patient, the indicia at the septum provides at least one identification of the port under x-ray or computer tomography imaging.

An embodiment may further comprise a port, comprising: a housing having a top opening; a resealable elastomer septum sealingly fitted to the top opening to establish a reservoir in the housing, the septum having a depression of a given configuration at a top surface whereinto a radiopaque insert having the given configuration is mounted; an outlet provided to a lower portion of the housing to establish a fluid path from the reservoir to outside of the housing; wherein the septum is adapted to be punctured by a cannula so that fluid may be input to or withdrawn from the reservoir; and wherein, when the port is implanted in a patient, the radiopaque insert provides an information bearing indicia viewable under x-ray or computer tomography imaging.

The insert may comprise a molded one piece radiopaque insert; and wherein after the insert is mounted into the depression of the elastomer, the top of the elastomer is covered by a silicone layer sealingly bonded to the elastomer to encapsulate the insert.

A catheter may be connected to the outlet to guide the fluid from the reservoir to a desired location within the patient.

It is also described a method of manufacturing a septum adapted to be used with a port, comprising the steps of: (a) molding an elastomeric septum to have at least one depression of a given configuration at a top surface thereof; (b) molding a radiopaque insert with the given configuration; (c) mounting the insert into the depression at the septum; and (d) covering the top surface of the septum with a top layer bondable to the septum.

Step (b) may further comprise the steps of: providing a mold having a mold cavity with the given configuration; injecting a radiopaque material into the mold cavity; solidifying the injected radiopaque material in the mold cavity; and extracting the solidified one piece radiopaque insert from the mold cavity. Step (d) may further comprise the step of: channeling a liquid silicone to the top of the septum to form the top layer. Step (a) may further comprise the step of: forming the depression of the given configuration as an indicia adapted to convey information to a viewer.

Another method of manufacturing a septum, comprises the steps of: (a) molding an elastomeric septum base to have at least one depression of a given configuration at its top surface; (b) molding a radiopaque septum top having a formation in the form of an insert having the given configuration hanging from its bottom surface; and (c) placing the septum top onto the septum base with the insert fittingly mounted to the depression at the septum base.

Step (b) may further comprise the steps of: impregnating a fluid silicone from which the septum top is formed with a radiopaque material; injecting the radiopaque material impregnated fluid silicone to a septum top mold; solidifying the injected fluid silicone into a one piece septum top with the insert integrally hanging from its bottom surface; and extracting the one piece radiopaque septum top from the septum top mold.

Step (a) may further comprises the step of: forming the depression to have a predetermined depth into the septum base from the top surface of the septum base so that the insert of the septum top is fully mounted into the depression when the septum top is placed on top of the septum base, the mounted insert providing a readable indicia to convey information to a viewer under x-ray or computer tomography imaging.

An embodiment may provide a port, comprising: a housing having a top opening; a resealable elastomer septum sealingly fitted to the top opening to establish a reservoir in the housing, an outlet provided to a lower portion of the housing to establish a fluid path between the reservoir and the outside of the housing; wherein the septum is adapted to be punctured by a cannula so that fluid may be input to or withdrawn from the reservoir; and wherein the septum comprises a one piece septum top bondingly secured to a septum base, the septum top being radiopaque and having a formation in the form of an insert of a given configuration hanging from its bottom surface, the septum base having a depression of the given configuration formed at its top surface so that when the septum top is placed onto the septum base, the insert hanging from the bottom surface of the septum top is fittingly mounted into the depression at the septum base; and wherein the insert mounted to the depression at the septum base provides an information bearing indicia viewable under x-ray or computer tomography imaging.

The septum top with the hanging insert may be formed as an integral one piece solidified radiopaque elastomer, and wherein the septum base is a one piece non-radiopaque elastomer.

Inasmuch as the present invention is subject to many variations, modifications and changes in detail, it is intended that all matter described throughout this specification and shown in the accompanying drawings be interpreted as illustrative only and not in a limiting sense.

## Claims

1. A septum (8) including a resealable elastomer septum base (8a) having a top surface, at least one depression (8d) of a given configuration formed at the top surface of said septum base (8a), said depression (8d) viewable by a viewer as an information bearing indicia, **characterised in that** the septum includes a molded solid one piece radiopaque insert (16, 16') having the given configuration fittingly mounted in said depression (8d), said radiopaque insert (16, 16') viewable under x-ray or computer tomography imaging.

2. The septum of claim 1, wherein said radiopaque insert (16) is extracted from a mold and is configured as a readable indicia to convey information about a port (2) the septum sealingly fits to.

3. The septum of claim 1, further **characterized in that** a top layer (18) covers the top surface of said septum base (8a) and said insert (16).

4. The septum of claim 3, wherein said top layer (18) comprises a silicone layer sealingly bonded to the top surface of said septum base (8a) to encapsulate said insert (16).

5. The septum of claim 1, wherein said depression (8d) is formed of one continuous cavity with a curved internal trench surface, and wherein said insert (16) is molded to have a flat top surface (16a) and an outwardly curved under surface (16b) so that said insert (16) may be fittingly mounted into said cavity formed by said depression (8d).

6. The septum of claim 1, wherein said septum is fitted to an implantable port (2) and wherein when the port (2) is implanted in a patient, said insert (16) provides a location whereby a clinician can access the port (2) under x-ray or computer tomography imaging.

7. The septum of claim 1, further **characterized in that** said insert (16') is a part of a top layer (38) and integrally hangs downwardly from the bottom surface (38b) of said top layer so that when said insert (16') fittingly mounts to said depression (8d), the top surface of said septum base (8a) is covered by said upper layer (38).

8. The septum of claim 7, wherein said top layer (38) and its integral insert (16') are molded from a liquid silicone impregnated with a radiopaque material so that both said top layer (38) and its integral insert (16') are radiopaque.

9. The septum of claim 1, wherein said insert (16) is molded from Barium Sulfate (BaSO4).

10. A port implantable into a patient, **characterised in that** the port includes a housing (10) having an opening and an outlet (10d), the opening being sealingly fitted with said septum (8) of any of the preceding claims to establish a reservoir in the housing (10), wherein said radiopaque insert (16) in said septum (8) is viewable under x-ray or computer tomography imaging to convey information about the port when the port (2) is implanted in a patient.

11. The port of claim 10, wherein when the port (2) is implanted in a patient, said insert (16) provides a location of the septum (8) under x-ray or computer tomography imaging whereby a clinician can access the reservoir in the housing (10) by piercing the septum (8) with a cannula.

## Patentansprüche

1. Septum (8), beinhaltend eine wiederverschließbare Elastomer-Septumbasis (8a), die eine Oberseite aufweist, wobei an der Oberseite der Septumbasis (8a) mindestens eine Vertiefung (8d) einer gegebenen Konfiguration gebildet ist, wobei die Vertiefung (8d) für einen Betrachter als eine mit Markierungen versehene Information sichtbar ist, **dadurch gekennzeichnet, dass** das Septum einen stabilen, geformten, einteiligen röntgendichten Einsatz (16, 16'), der die gegebene Konfiguration aufweist, beinhaltet, der passend in der Vertiefung (8d) angebracht ist, wobei der röntgendichte Einsatz (16, 16') bei Röntgen- oder Computertomographie-Bildgebung sichtbar ist.

2. Septum nach Anspruch 1, wobei der röntgendichte Einsatz (16) aus einer Form extrahiert ist und als eine lesbare Markierung konfiguriert ist, um Informationen über einen Anschluss (2), an den das Septum dichtend passt, zu übermitteln.

3. Septum nach Anspruch 1, ferner **dadurch gekennzeichnet, dass** eine Deckschicht (18) die Oberseite der Septumbasis (8a) und des Einsatzes (16) bedeckt.

4. Septum nach Anspruch 3, wobei die Deckschicht (18) eine Silikonschicht umfasst, die mit der Oberseite der Septumbasis (8a) dicht verbunden ist, um den Einsatz (16) zu verkapseln.

5. Septum nach Anspruch 1, wobei die Vertiefung (8d) aus einem kontinuierlichen Hohlraum mit einer gekrümmten inneren Rinnenoberfläche gebildet ist, und wobei der Einsatz (16) so geformt ist, dass er eine flache Oberseite (16a) und eine nach außen gekrümmte Unterseite (16b) aufweist, sodass der Einsatz (16) passend in dem durch die Vertiefung (8d) gebildeten Hohlraum angebracht werden kann.

6. Septum nach Anspruch 1, wobei das Septum an einem implantierbaren Anschluss (2) montiert ist, und wobei, wenn der Anschluss (2) in einem Patienten implantiert ist, der Einsatz (16) einen Standort bereitstellt, mittels dessen ein Klinikarzt unter Röntgen- oder Computertomographie-Bildgebung auf den Anschluss (2) zugreifen kann.

7. Septum nach Anspruch 1, ferner **dadurch gekennzeichnet, dass** der Einsatz (16') Teil einer Deckschicht (38) ist und von der Unterseite (38b) der Deckschicht integral nach unten hängt, sodass, wenn der Einsatz (16') passend in der Vertiefung (8d) angebracht ist, die Oberseite der Septumbasis (8a) mit der Deckschicht (38) bedeckt ist.

8. Septum nach Anspruch 7, wobei die Deckschicht (38) und ihr integraler Einsatz (16') aus einem Flüssigsilikon geformt sind, das mit einem röntgendichten Material imprägniert ist, sodass sowohl die Deckschicht (38) als auch ihr integraler Einsatz (16') röntgendicht sind.

9. Septum nach Anspruch 1, wobei der Einsatz (16) aus Bariumsulfat (BaSO4) geformt ist.

10. Anschluss, der einem Patienten implantiert werden kann, **dadurch gekennzeichnet, dass** der Anschluss ein Gehäuse (10) beinhaltet, dass eine Öffnung und einen Auslass (10d) aufweist, wobei die Öffnung dichtend mit dem Septum (8) nach einem der vorhergehenden Ansprüche bestückt ist, um ein Reservoir in dem Gehäuse (10) zu schaffen, wobei der röntgendichte Einsatz (16) in dem Septum (8) unter Röntgen- oder Computertomographie-Bildgebung sichtbar ist, um Informationen über den Anschluss zu übermitteln, wenn der Anschluss (2) in einem Patienten implantiert ist.

11. Septum nach Anspruch 10, wobei der Anschluss (2) in einem Patienten implantiert ist, wobei der Einsatz (16) unter Röntgen- oder Computertomographie-Bildgebung einen Standort des Septums (8) bereitstellt, mittels dessen ein Klinikarzt durch Durchstechen des Septums (8) auf das Reservoir in dem Gehäuse (10) zugreifen kann.

## Revendications

1. Septum (8) incluant une base de septum en élastomère refermable (8a) ayant une surface supérieure, au moins une dépression (8d) d'une configuration donnée formée à la surface supérieure de ladite base de septum (8a), ladite dépression (8d) pouvant être vue par un observateur comme une information portant des indices, **caractérisée en ce que** le septum inclut un insert monobloc radio-opaque solide moulé (16, 16') ayant une configuration donnée montée sur ladite dépression (8d), ledit insert radio-opaque (16, 16') visible sous radiographie ou imagerie par tomodensitométrie.

2. Septum selon la revendication 1, dans lequel ledit insert radio-opaque (16) est extrait d'un moule et est configuré comme un indice lisible pour transmettre des informations sur un orifice (2) auquel le septum s'adapte hermétiquement.

3. Septum selon la revendication 1, **caractérisé en outre en ce qu'**une couche supérieure (18) couvre la surface supérieure de ladite base de septum (8a) et dudit insert (16).

4. Septum selon la revendication 3, dans lequel ladite couche supérieure (18) comprend une couche de silicone scellée hermétiquement à la surface supérieure de ladite base de septum (8a) pour encapsuler ledit insert (16).

5. Septum selon la revendication 1, dans lequel ladite dépression (8d) est formée d'une cavité continue avec une surface de tranchée interne incurvée, et dans lequel ledit insert (16) est moulé pour avoir une surface supérieure plane (16a) et une surface inférieure incurvée vers l'extérieur (16b) de telle sorte que ledit insert (16) puisse être monté de manière ajustée dans ladite cavité formée par ladite dépression (8d) .

6. Septum selon la revendication 1, dans lequel ledit septum est monté sur un port implantable (2) et dans lequel, lorsque le port (2) est implanté chez un patient, ledit insert (16) fournit un emplacement où un clinicien peut accéder au port (2) sous radiographie ou imagerie par tomodensitométrie.

7. Septum selon la revendication 1, **caractérisé en outre en ce que** ledit insert (16') fait partie d'une couche supérieure (38) et est suspendu intégralement vers le bas à partir de la surface inférieure (38b) de ladite couche supérieure de sorte que lorsque ledit insert (16') est monté de manière ajustée sur ladite dépression (8d), la surface supérieure de ladite base de septum (8a) est couverte par ladite couche supérieure (38).

8. Septum selon la revendication 7, dans lequel ladite couche supérieure (38) et son insert intégral (16') sont moulés à partir d'une silicone liquide imprégné d'un matériau radio-opaque de sorte que ladite couche supérieure (38) et son insert intégral (16') soient radio-opaques.

9. Septum selon la revendication 1, dans lequel ledit insert (16) est moulé à partir de sulfate de baryum (BaSO₄).

10. Port implantable dans un patient, **caractérisé en ce que** le port inclut un logement (10) ayant une ouverture et une sortie (10d), l'ouverture étant ajustée de manière étanche avec ledit septum (8) selon l'une quelconque des revendications précédentes pour établir un réservoir dans le logement (10), dans lequel ledit insert radio-opaque (16) dans ledit septum (8) est visible sous radiographie ou imagerie par tomodensitométrie dans le but de transmettre des informations sur le port lorsque le port (2) est implanté chez un patient.

11. Port selon la revendication 10, dans lequel, lorsque le port (2) est implanté chez un patient, ledit insert (16) fournit un emplacement du septum (8) sous radiographie ou imagerie par tomodensitométrie par lequel un clinicien peut accéder au réservoir dans le logement (10) en perçant le septum (8) avec une canule.
